# EUROPEAN PATENT APPLICATION

(11) **EP 1 935 496 A1**
(43) Date of publication of application: **25.06.2008**
(21) Application number: 07150382.5
(22) Date of filing: 21.12.2007
(51) Int. Cl.: B01L 3/00, C12Q 1/68, B01J 19/00, G01N 33/543

(54) **Device and/or method for the detection of amplified nucleotides sequences on micro-arrays**

(30) Priority: 22.12.2006 EP 06127163
(71) Applicant: Eppendorf Array Technologies SA, 5000 Namur (BE)
(72) Inventor: Koehn, Heinz, DE-22339, Hamburg (DE); Remacle, José, B-5020, Malonne (BE); Seippel, Martin, DE-22391, Hamburg (DE); Alexandre, Isabelle, B-5340, Haltinne (BE)
(74) Representative: pronovem

(57) **Abstract**

A device for a detection of a nucleotide sequence of an organism, said device comprising:
- at least one amplification chamber (1) for an amplification of the nucleotide sequence into amplified target nucleotide sequences, the said chamber (1) having an inlet port (11) for an introduction into the amplification chamber of an amplification solution (1) comprising said nucleotide sequence and reagents for nucleotide sequence amplification, having a first outlet channel (13) and is made of solid material resistant to at least 90°C;
- at least one reagent chamber (2), said reagent chamber (2) having a second inlet port (12) for introduction of the reagent and a second outlet channel (14);
- a detection chamber (3) having fixed upon one of its solid support surface (15) at least one capture nucleotide probe being immobilized in discrete regions of said surface (15) to form a micro-array (16), said capture nucleotide probe being capable to bind by complementary base pairing with the amplified target nucleotide sequence or fragments thereof, wherein said surface (15) of the detection chamber (3) having fixed the capture nucleotide probe is maintained flat at temperature higher than 50°C, wherein the flatness tolerance of said surface (15) is less than 800 nm, when heated at 50°C and wherein said surface (15) has a light transmittance higher than 60% at wavelength between 400 and 600 nm and,
- a fragmentation chamber (4) for a fragmentation of amplified target nucleotide sequences into fragments, the said fragmentation chamber being connected to the amplification chamber (1) and to the reagent chamber (2) by the first and second outlet channels (13, 14) and being connected to the detection chamber (3) by a third outlet channel (19) and wherein said first and second channels (13, 14) are located at a different level than the third channel (19).

## Description

### Field of the invention

The present invention relates to a device and/or a method for performing (upon the same device) a genetic amplification, preferably a polymerase chain reaction (PCR) of a nucleotide sequence from an organism, an addition of a reagent, an hybridization step on a micro-array and a detection of this nucleotide sequence.

The invention is well suited for a simultaneous identification and/or quantification of multiple mutations or single nucleotide polymorphisms (SNPs) present simultaneously in the same gene nucleotide sequence or same organism genome. The present invention is well adapted for diagnostic and automated assay.

### Description of the related art

The disclosed nucleotide molecule detection method offers the advantages of speed, simplicity and multiplexing over prior methods for detecting multiple amplified nucleic acids. Nucleic acid detection techniques in general are very useful in medical diagnostic assays.

The sensitivity and specificity of nucleic acids detection methods were greatly improved by the genetic amplification techniques such as the polymerase chain reaction (PCR). PCR is a process for amplifying nucleic acids and involves the use of two oligonucleotide primers, an agent for polymerization, a target nucleic acid template, and successive cycles of denaturation of nucleic acid and annealing and extension of the primers to produce a large number of copies of a particular nucleic acid segment. With this method, segments of single copy genomic DNA can be amplified more than 10 million fold with very high specificity and fidelity.

Methods for detecting PCR amplified products are described in U.S. Pat. No. 4,683,195. These methods require an oligonucleotide probe capable of hybridizing with the amplified target nucleic acid and require separate steps of amplification, capture, and detection and generally require several hours to complete.

Due to the enormous amplification obtained with the PCR process, small levels of DNA carryover from samples with high DNA content, from positive control templates, or from previous amplifications, can result in PCR amplified products even in the absence of purposefully added template DNA. Contamination by amplicon pipeting due to the formation of aerosols is a main issue for laboratories performing the same PCR routine analysis. The possibility of introducing contaminating DNA to a sample increases as the number of handling steps required for sample preparation, processing, and analysis increased and it would be preferable to minimize sample handling for their detection and quantification, particularly after the amplification reaction is complete and still especially when multiple DNA or RNA sequences have to be differentially detected and/or quantified.

### Aims of the invention

A first aim of the present invention is to provide a closed device and a method for PCR amplification that do not present the drawbacks of the state of the art and is suitable for an efficient, rapid and automated identification and/or quantification of amplified sequences on micro-array.

Another aim of the invention is to provide a device and a method that simplifies and reduces the costs of a (PCR) amplification and detection of amplified sequence(s).

### Summary of the invention

The present invention concerns a device for a detection (and/or Quantification) of a nucleotide sequence of an organism, this device comprising the following elements:
- at least one amplification chamber for an amplification of the nucleotide sequence (being amplified into target nucleotide sequences), the said chamber possibly containing an amplification solution, and the said chamber having an inlet port for an introduction of the amplification solution in the amplification chamber, this solution comprising the nucleotide sequence to be detected and reagents for nucleotide sequence amplification, this chamber having a first outlet channel and is made of solid material resistant to high temperature, i.e. at least 90°C, preferably to at least 95°C;
- at least one reagent chamber (possibly containing a reagent), this reagent chamber having a second inlet port for introduction of a reagent and having a second outlet channel;
- a detection chamber having fixed upon one of its solid support surface at least one capture (nucleotide) probe (or molecule) being immobilized in discrete regions of this surface to form a micro-array, the capture (nucleotide) probe is capable to bind (by complementary base pairing [hybridization]) with the amplified target nucleotide sequences or a fragment thereof and,
- a fragmentation chamber (4) for a fragmentation of amplified target nucleotide sequences into fragments, the said fragmentation chamber (4) being connected to the amplification chamber (1) and to the reagent chamber (2) by the first and second outlet channels (13, 14) and being connected to the detection chamber (3) by a third outlet channel (19) and wherein said first and second channels (13, 14) are located at a different level than the third channel (19).
The present is also related to a method for the detection (identification) and /or quantification of a nucleotide sequence which comprises the use of this device.

According to the invention, the amplification chamber is made of solid material resistant to at least 90°C which means that the material is kept solid or rigid (it is not melting) and the shape of the material is kept and preferably, there is no chemical modification of the material. According to the invention, the surface of the detection chamber having fixed the capture nucleotide probe is maintained flat at temperature higher than 50°C, preferably higher than 60°C and even more preferably higher than 70°C.

According to the invention, the flatness tolerance of this surface is less than 800 microns, preferably less than 400 microns, even more preferably less than 100 microns when heated at 50°C and this surface has a light transmittance higher than 60% (preferably higher than 80% and even higher than 90%) at wavelength between 400 and 600 nanometers (nm).

### Description of the drawings

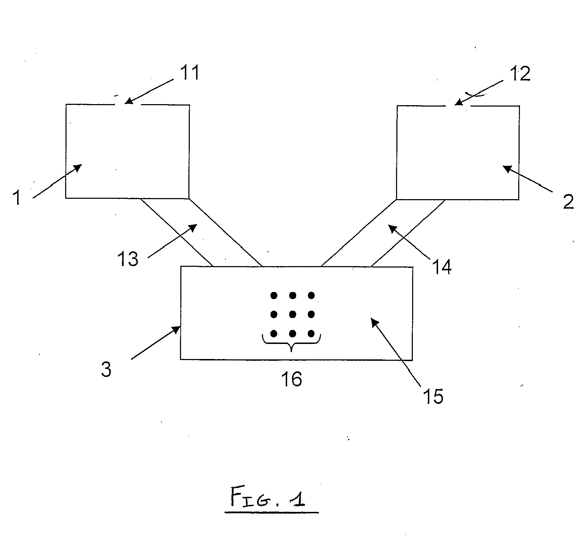

Figure 1 represents a Flowchart of the different steps performed in the method of the invention with the device 10 of the invention for a detection of a nucleotide sequence of an organism. In a first step, a nucleotide sequence and reagents for nucleotide sequence amplification are introduced into an amplification chamber 1 through inlet port 11 for a amplification of this nucleotide sequences into (possibly labelled) amplified target nucleotide sequences. In a reagent chamber 2, a reagent is introduced through inlet port 12. The reagent is preferably a salt solution having a higher ionic strength than the PCR solution in order to make it suitable for hybridization. An automated amplification (PCR) process is performed in the amplification chamber 1 and (possibly labelled) amplified target sequences (amplicons) are generated. After (PCR) amplification, the contents of the amplification and the reagent chambers are introduced into a detection chamber 3 through two outlet channels 13, 14 by flipping the device 10. The mixing of the two solutions in the detection chamber 3 constitutes the second step. The detection chamber 3 has fixed upon one of its solid support surface 15 a micro-array 16 of capture probes and hybridization is performed in a third step. The reading of the signal resulting from the binding between said labelled amplicons and the capture probes of the micro-array is preferably performed in the absence of solution on the micro-array that is easily obtained by flipping the device 10 in order to transfer the content of the detection chamber 3 back to the amplification chamber 1 and/or to the reagent chamber 2.

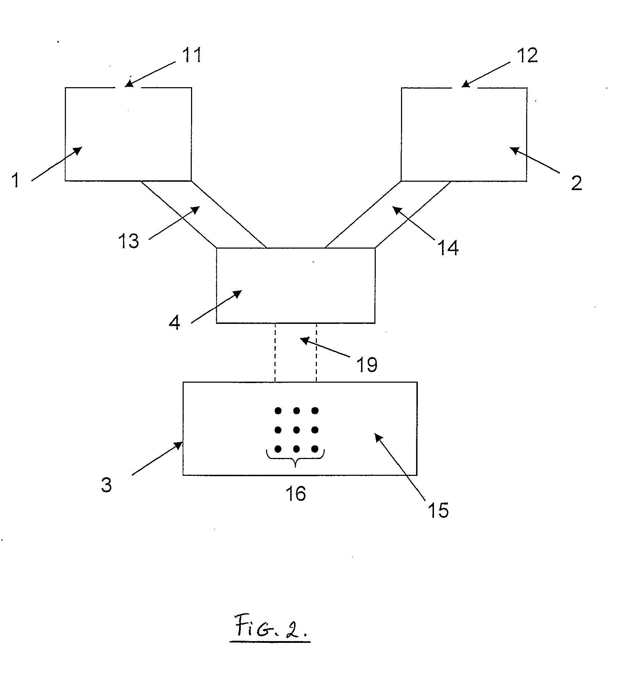

Figure 2 represents a Flowchart of different steps performed in the method of the invention with the device 10 of the invention for a detection of a nucleotide sequence of an organism. In a first step, a nucleotide sequence and reagents for nucleotide sequence amplification are introduced into an amplification chamber 1 through inlet port 11. In a reagent chamber 2, a reagent (DNase) for fragmenting the (labelled) amplicons into fragments is introduced through inlet port 12. An automated PCR process is performed in the amplification chamber 1 and (labelled) amplicons are generated. After the PCR, the contents of the amplification and reagent chambers 1,2 are introduced into a fragmentation chamber 4 through two channels 13, 14 by flipping the device 10. In a second step, the (labelled) amplicons are cut into (labelled) fragments in this fragmentation chamber 4. In a third step, these fragmented (labelled) amplicons (fragments) are introduced through third channel 19 (by flipping the device 10) into a detection chamber 3 having fixed upon one of its solid support surface 15 a micro-array 16 of capture probe(s) and hybridization is performed. The reading of the signal resulting from the binding between said fragmented (labelled) amplicons (fragments) and the capture probe(s) of the micro-array 16 is preferably performed in the absence of solution on the micro-array 16 that is easily obtained by flipping the device 16 in order to transfer the content of the detection chamber 3 into the fragmentation chamber 4. The device is made in such a manner that the amplification and reagent channels 13,14 are located at a different level or plan than the detection channel 19.

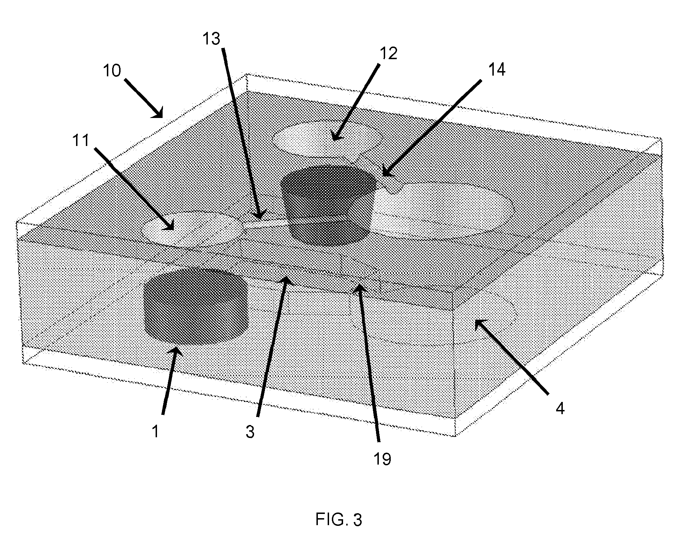

Figure 3 represents a 3D view of the device 10 according to the invention having four chambers as provided in the flowchart of figure 2.

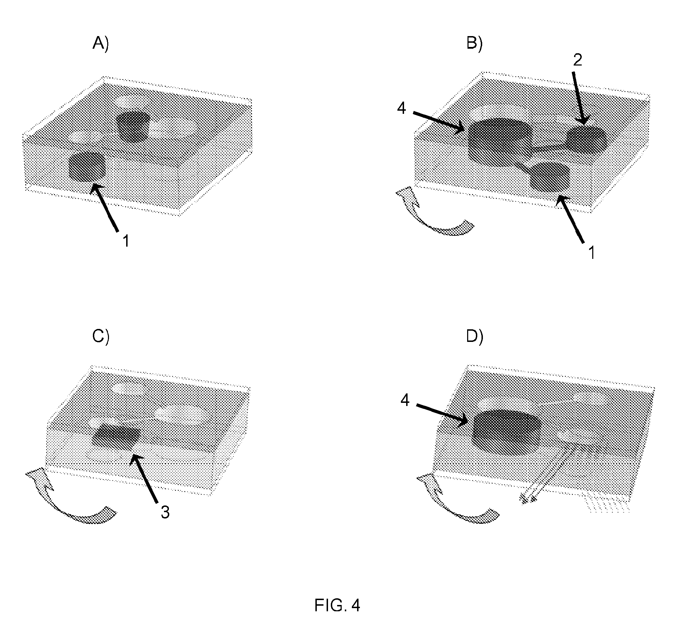

Figure 4a to figure 4d are schematic representations of the four steps of the method of the invention with the device of the invention as provided in figure 2.
Step1 (fig. 4a) An amplification chamber 1 contains a solution comprising a nucleotide sequence and reagents for nucleotide sequence amplification into amplified nucleotide sequences. The amplification chamber 1 has an inlet port 11 for introducing the solution and a first outlet channel 13. A reagent chamber 2 contains a reagent. The reagent chamber 2 has an inlet port 12 for introducing the reagent and a second outlet channel 14. In this first step, a PCR is performed in the amplification chamber 1.
Step 2 (fig. 4b) After the PCR, the content of amplification 1 and reagent 2 chambers are introduced into a fragmentation chamber 4, through first and second outlet channels 13,14 by flipping the device 10. The amplified nucleotide sequences are cut into fragments in this fragmentation chamber.
Step 3 (fig.4c) The fragmented amplified nucleotide sequences (fragments) are introduced by flipping the device 10 into a detection chamber 3 having fixed upon one of its surface a micro-array of capture probes and hybridization is performed.
Step 4 (fig. 4d) The reading of the signal resulting from the binding between the fragmented amplified nucleotide sequences (fragments) and the capture probes of the micro-array 16 is performed. The reading is preferably performed in the absence of solution on the micro-array 16 that is easily obtained by flipping the device 10 in order to transfer the content of the detection chamber 3 back to the fragmentation chamber 4.

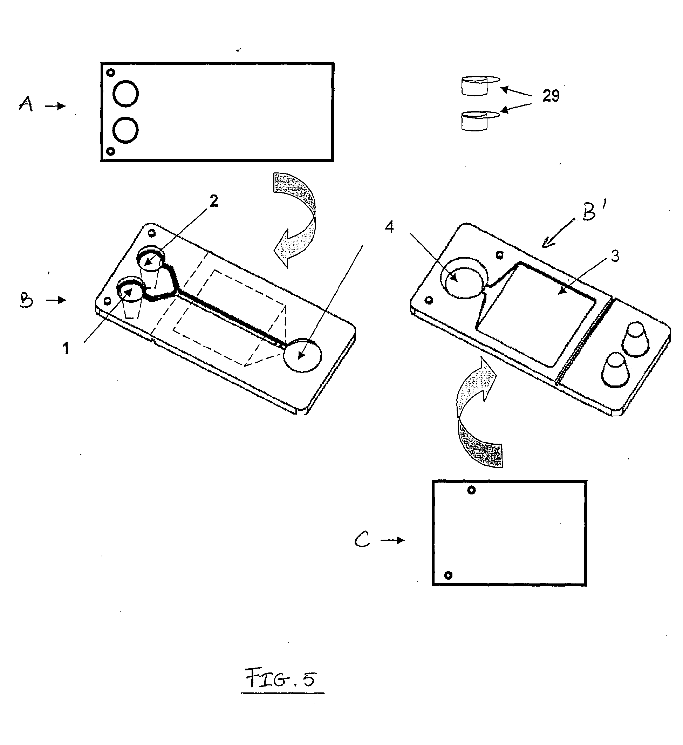

Figure 5 is a schematic representation of the different parts of the device 10 following one preferred embodiment of the invention. The device 10 is made of three parts which are welded together. The first part is a coverslip holding two holes fitting with top of two chambers 1,2 of a second part, said coverslip also holding two small holes at two corners for positioning onto the upper side of second part. The aim of this part is to seal the upper side of the second part. The second part is the central part of the device 10. It is made of two sides (upper and lower) carrying elements which are in different plans. The upper side of the second part comprises two chambers (amplification chamber 1, reagent chamber 2), each chamber been connected to a channel. The two channels connect together to make one channel crossing the whole device to reach the fragmentation chamber 4. The fragmentation chamber 4 is connected to an hybridization (detection) chamber 3 being located in another plan (lower side). The third part is a coverslip holding two holes for positioning onto the lower side of the second part. The aim of this part is to seal the fragmentation chamber and the hybridization chamber. These chambers 1, 2 are closed by tube caps 29 or a sealing film before performing a microfluidic reaction.

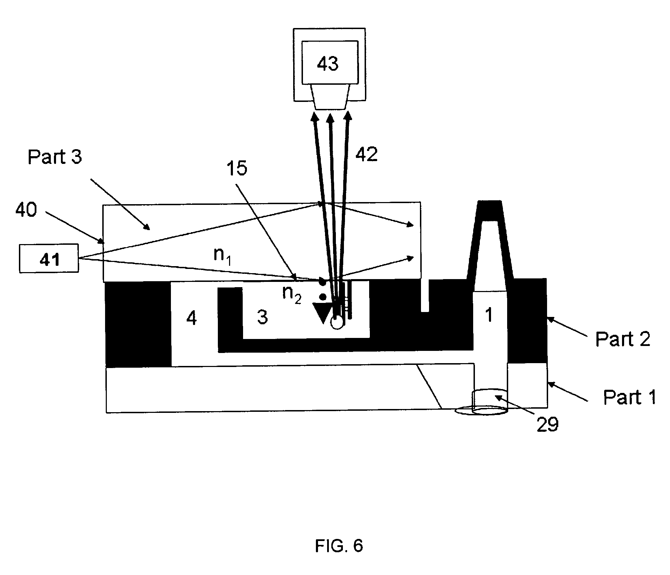

Figure 6 is a schematic representation of of a side view of the device 10 for performing amplification (PCR) and detection of labeled amplicons on immobilized capture nucleotide probes using evanescent field, which is depicted as a black triangular arrow with a black dot shaft pointing down from the device part C. Part C has a first refractive index (n₁) and the solution containing the labeled amplicons has a second refractive index (n₂). The first refractive index (n₁) is higher than the second refractive index (n₂) to create an evanescent field. The detection chamber 3 has preferably black side walls 5'. The detection chamber 3 is closed on its top by Part C and the amplification chamber 1 is closed at its bottom by part A. A side 40 of Part C is illuminated by a light source 41, preferably a diverging laser beam or light emitting diode. The emitted light 42 passes through Part C of the device 10 and is collected by a detector 43.

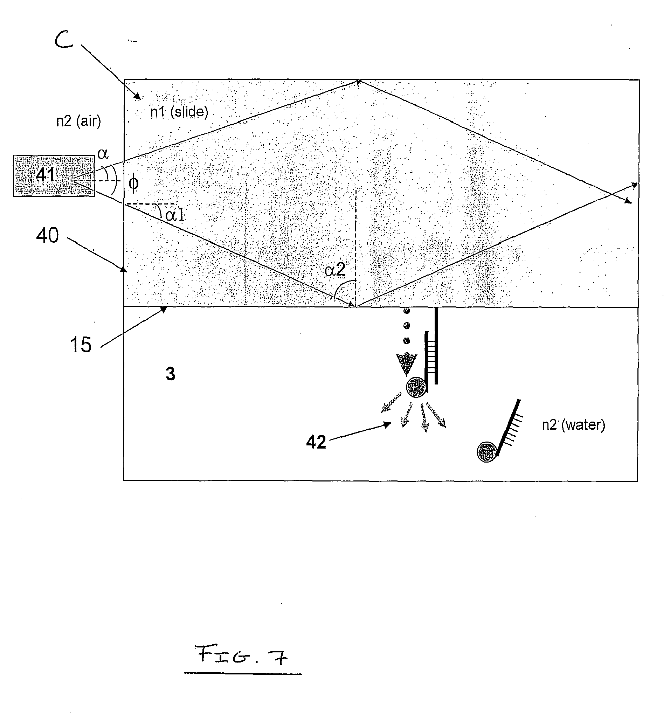

Figure 7 is an enlargement of the interface between the solution containing the labeled amplicons in the detection chamber 3 and the surface of the device 10 (Part C) having fixed the capture nucleotide probes as represented in Figure 6. The angle α2 represents the incident angle that allows total internal reflection and creates an evanescent field. Each optical fiber (of the light source 41) faces the side 40 of Part C of the device 10 and couples light through a divergent diffuser at a restricted angle (φ).

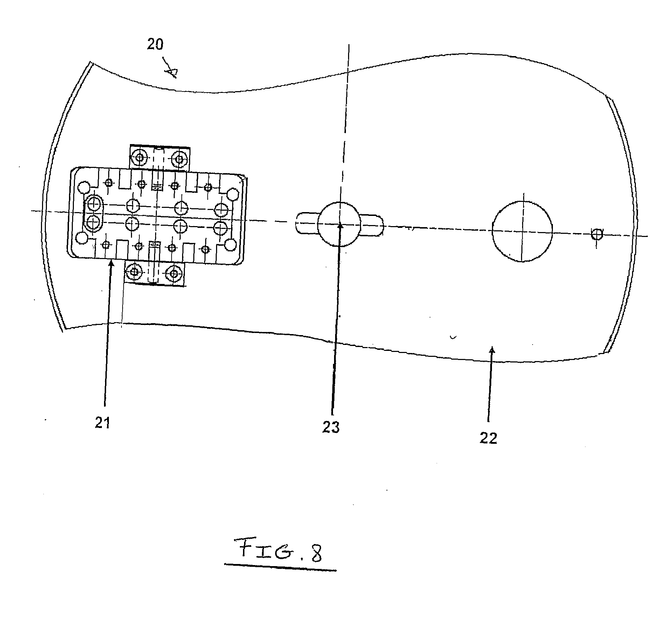

Figure 8 illustrates the device 10 as represented in figure 5 placed in a centrifugation rotor 20. The device is positioned in a centrifugation adaptor 21 that may be positioned in the centrifugation rotor 20 at a variable angle relative to the direction of the applied centrifugal force to facilitate solution transfer from one chamber to another chamber. To transfer solution from one or two chamber (s) 1 and 2 to another chamber 4 (which are in the same focal plane), the adaptor 21 is positioned at the surface of the rotor 22. Chambers 1 and 2 of the device are positioned close to the center 23 of the rotor 20 while the other chamber 4 is positioned far from this center. To transfer solution from chamber 4 to chamber 3 (which are in different focal plane), the adaptor 21 is positioned at an angle of 80° relative to the direction (F) of the applied centrifugal force. Chamber 4 is positioned close to the center 23 of the rotor 20 (upper position) while chamber 3 is positioned far from the center of the rotor 20 (down position).

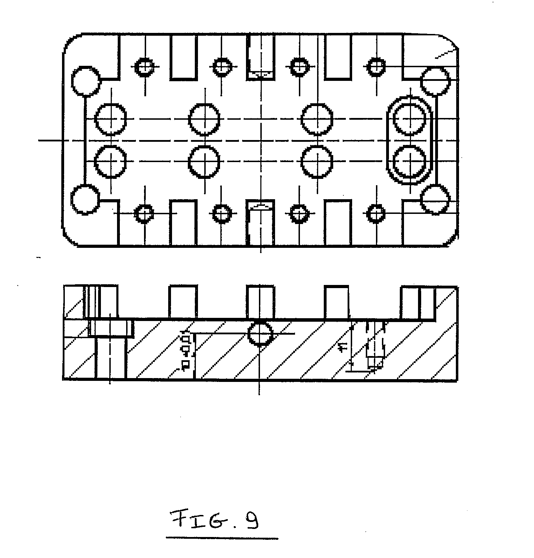

Figure 9 illustrates a centrifugation adaptor 21 as presented in figure 8. The upper drawing represents a top view of the adaptor 21 and the lower drawing a side view.

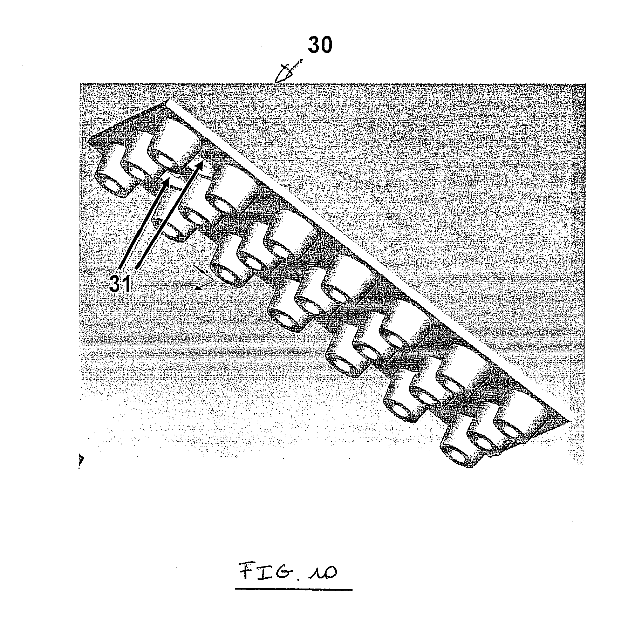

Figure 10 illustrates a thermic adaptor 30 used during the PCR. The two chambers 1 and 2 of the device 10 are positioned into the two holes 31 of the thermic adaptor 30 and then the adaptor is inserted into a 96-wells thermoblock 32 of a conventional PCR thermocycler.

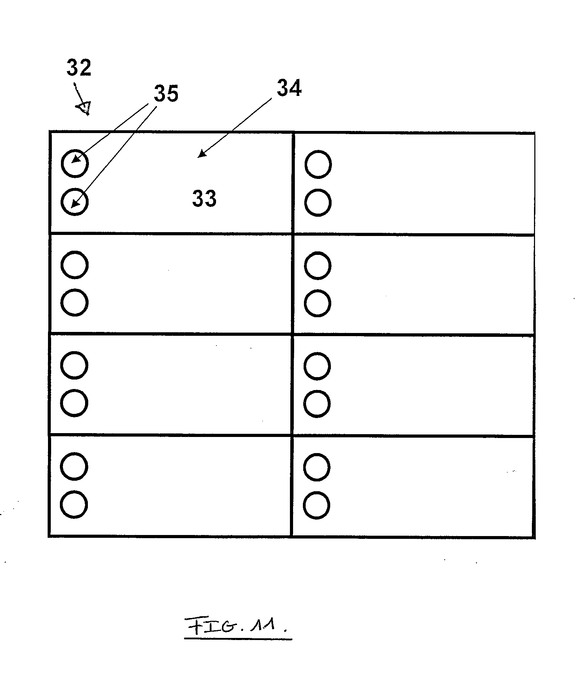

Figure 11 illustrates a thermoblock 32 specifically designed to receive twelve devices 10 of the invention. The thermoblock 32 is made of twelve segments 33, each segment being adapted to receive one device 10. Each segment comprises a flat surface 34 and two cavities 35 being adapted to receive the amplification and reagent chambers 1,2 of the device 10.

### Definitions

Unless otherwise defined, all technical and scientific terms used herein have the same meaning as commonly understood by one person ordinary skilled in the art to which this invention belongs.

The terms "nucleotide sequence, micro-array, target (and capture) nucleotide sequence, bind substantially, hybridizing specifically to, background, quantifying" are as described in WO97/27317, which is incorporated herein by way of reference.

The terms "nucleotide triphosphate, nucleotide, primer sequence" are those described in the European patent application EP1096024 incorporated herein by reference.

The term "gene" means fundamental physical and functional unit of heredity, which carries information from one generation to the next; a segment of DNA located in a specific site on a chromosome that encode a specific functional product. The DNA segment is composed of transcribed region and a regulatory sequence that makes transcription possible (regions preceding and following the coding DNA as well as introns between the exons).
The term "locus" means the position of the single nucleotide polymorphism (SNP) upon the sequence of the gene.

"Homologous sequences" mean nucleotide sequences having a percentage of nucleotides identical at corresponding positions which is higher than in purely random alignments. Two sequences are considered as homologous when they show between them a minimum of homology (or sequence identity) defined as the percentage of identical nucleotides found at each position compared to the total nucleotides, after the sequences have been optimally aligned taking into account additions or deletions (like gaps) in one of the two sequences to be compared. The degree of homology (or sequence identity) can vary a lot as homologous sequences may be homologous only in one part, a few parts or portions or all along their sequences. Nucleotide sequences differing by only one base are sequences highly homologous and qualified as single nucleotide polymorphisms (SNPs). The parts or portions of the sequences that are identical in both sequences are said conserved. Protein domains which present a conserved three dimensional structure are usually coded by homologous sequences and even often by a unique exon. The sequences showing a high degree of invariance in their sequences are said to be highly conserved and they present a high degree of homology.

Methods of alignment of sequences are based on local homology algorithms which have been computerised and are available as for example (but not limited to) Clustal®, (Intelligenetics, Mountain Views, California), or GAP®, BESTFIT®, FASTA® and TFASTA® (Wisconsin Genetics Software Package, Genetics Computer Group Madison, Wisconsin, USA) or Boxshade®.

The term "consensus sequence" is a sequence determined after alignment of the several homologous sequences to be considered (calculated as the base which is the most commonly found at each position in the compared, aligned, homologous sequences). The consensus sequence represents a sort of «average» sequence which is as close as possible from all the compared sequences. For high homologous sequences or if the consensus sequence is long enough and the reaction conditions are not too stringent, it can bind to all the homologous sequences. This is especially useful for an amplification of homologous sequences with the same primers called, consensus primers. Experimentally, the consensus sequence calculated from the programs above can be adapted in order to obtain such property.

"Micro-arrays and arrays" mean solid supports on which single capture probes or capture probes species are immobilized in order to be able to bind to the given specific protein or target. The most common arrays are composed of single capture probes species being present in predetermined locations of a solid support being or not a substrate for their binding. The micro-array is preferentially composed of spots of capture probes deposited at a given location on the surface or within the solid support or on the substrate covering the solid support. However, capture probes can be present on the solid support in various forms being but not limited to spots. One particular form of application of micro-array is the presence of capture probes in wells having either one of several different capture probes per well and being part of the same support. Advantageously, micro-arrays of capture probes are also provided on different supports as long as these different supports contain specific capture probes and may be distinguished from each other in order to be able to allow a quantification of a specific target sequence. This can be achieved by using a mixture of beads having particular features and being able to be recognized from each other in order to quantify the bound molecules.

The terms "capture probe" relate to molecules capable to specifically bind to a given polynucleotide or polypeptide. Preferably, polynucleotide binding is obtained through base pairing between two polynucleotides one being the immobilized capture probe or capture sequence and the other one being the target molecule (sequence) to be detected.

The term "single capture probe species" is a composition of related (poly)nucleotides for the detection of a given nucleotide sequence by base pairing hybridization.

Polynucleotides are synthesized either chemically or enzymatically or purified from samples. However, the synthesis or purification is not always perfect and the capture probe can be slightly contaminated by other related molecules like shorter polynucleotides.

Essential characteristic of one capture species for the invention is that the overall species can be used for capture of a given target molecule, preferably a given target nucleotide sequence.

The term "signal resulting from a specific binding at a specific location" means a detection and possibly a quantification of a single hybridization event between complementary nucleotide sequences at the specific localized area (location) of a fixed capture sequence of the solid support surface (or inside the solid support). A complementary hybridization can be detected and possibly quantified by a (fluorescent, colorimetric, etc.) label introduced in the sequence of the target sequence or at the extremity of the target sequence (preferably during the copy or amplification step) or by any of a number of means well known to those skilled in the art, such as detailed in WO 99/32660, which is incorporated herein by way of reference.

The term "different level" means that in a 3D structure of the device of the invention, the focal plan of a structure (first outlet channel) is different from the focal plan of another structure (i.e. third outlet channel).
The term "microfluidic" means microminiaturized devices with chambers and tunnels or channels for the containment and flow of fluids. Microfluidic devices are used in the present invention for the detection of a nucleotide sequence of an organism contained in a biological sample or body fluid (e.g. blood, saliva, urine) for medical or research purposes.
The term "fragments" refers to a portion of an amplified target nucleotide sequence cutted in the fragmentation chamber and corresponding to an (oligonucleotide) sequences having preferably between about 10 and about 150 nucleotides, preferably between about 20 and about 100 nucleotides, more preferably between about 25 and about 65 nucleotides, more specifically between about 30 and about 50 nucleotides.

### Detailed description of the invention

The present invention proposed a full and closed device used in a detection (and/or quantification) method to detect (and quantify) nucleotide sequence(s) being present in low amount by a series of three manipulations (i.e. amplification, mixing with a reagent and hybridization on a micro-array), each one having specific constraints. The detection, identification and/or quantification method steps of said nucleotide sequence is also specific due to the binding to specific capture molecule and the major method steps are performed without opening the device and without external input of liquid (during these method steps) and by simple physical handling of the device of the invention.

The present invention provides a closed device for endpoint measurement of PCR product on a micro-array and is particularly suited for method that allows determination of a presence or an absence of a particular base (mutated base) in a locus of a genetic sequence by examining a signal obtained in a particular location of an micro-array which serves to question (to detect and/or quantify) for the presence or not of the (mutated) base in a particular locus (SNPs).

To reach these objectives, the device 10 comprises at least three chambers 1,2,3 for performing the three steps (major steps of the detection and/or quantification method of the invention) of amplification of a nucleotide sequence , treatment of the amplified target nucleotide sequence and hybridization-detection of the amplified target nucleotide sequence on a micro-array. The flowchart of the method steps performed in the device is illustrated in figure 1.

The chamber 1 is an amplification chamber and is used to perform the amplification (step 1). This chamber 1 has an inlet port 11 for the introduction of a solution in said chamber 1 and a first outlet channel 13. This solution comprises a nucleotide sequence (possibly purified by one or more extraction and/or purification steps) to be detected and reagents for nucleotide sequence amplification of this (initial and possibly purified) sequence amplified into target nucleotide sequences (amplicons). Once the solution has been introduced into the chamber, the inlet port 11 is preferably sealed wit an adhesive, a cap 29 or any other suitable element.

The reagent chamber 2 allows to store reagents. This reagent chamber 2 has an inlet port 12 for the introduction of a reagent and a second outlet channel 14. Once the reagent has been introduced into the chamber, the inlet port 12 is preferably sealed with an adhesive, a cap 29 or any other suitable element.

The detection chamber 3 allows to perform a treatment of the amplified target nucleotide sequences (step 2) and hybridization on micro-array (step 3). The detection chamber 3 is connected to the amplification chamber 1 and to the reagent chamber 2 by a first outlet channel 13 and a second outlet channel 14. The detection chamber 3 has fixed upon one of its solid support surface 15 a micro-array 16 comprising at least one capture nucleotide molecule being immobilized in discrete regions of said surface, the capture nucleotide molecule being capable to bind by complementary base pairing (hybridization) with corresponding amplified target nucleotide sequence. The present invention concerns also a rapid, efficient and automatisable method for a detection (identification) and/or quantification of a nucleotide sequence which comprises various (and consecutive) steps of amplification, detection (and/or quantification) and possibly fragmentation of a nucleotide sequence that are performed upon the device of the invention as described hereafter.

In a preferred embodiment, for an advantageous and efficient detection and/or quantification the surface 15 of the detection chamber 3 having fixed the capture nucleotide molecule present specific physical characteristics such as: the surface is preferably maintained flat at temperature higher than about 50°C, preferably higher than about 60°C, even preferably higher than about 70°C and even more preferably higher than 95°C.
Preferably, the flatness tolerance of this surface 15 is less than about 800 microns, preferably less than about 400 microns and even more preferably less than about 100 microns.

In a preferred embodiment, the surface 15 of the detection chamber 3 suitable for an efficient detection by light, has a light transmittance at the wavelength used for a detection higher than 60% and even higher than 80%.

The device also comprises connective circuit for transfer of the liquid from one chamber to the other. To perform the treatment of the amplified DNA, the contents of amplification chamber 1 and reagent chamber 2 are introduced into the detection chamber 3 through the two outlet channels 13 and 14.

In a preferred embodiment, the reagent (possibly present in the reagent chamber 2) is a reagent solution having an ionic strength different from the amplification solution of the amplification chamber 1. Preferably, the reagent increases the hybridization rate on micro-array. In a preferred embodiment the reagent is a salt solution for changing the ionic strength of the solution contained in the amplification chamber 1. The salt concentration is preferably comprised between about 25 and about 300 mM. The preferred salts are: potassium glutamate, potassium chloride, sodium citrate and sodium chloride.

In another embodiment, the nucleotide amplification is preferably a PCR and the amplification solution contains a thermostable DNA polymerase enzyme that is active at a concentration in salt comprised between about 25 and about 300 mM. The preferred salts are: potassium glutamate, potassium chloride and sodium chloride. The polymerase enzyme is preferably a *Thermus aquaticus* DNA polymerase enzyme and more preferably the Topo Taq DNA polymerase. Thermostable means which still retains at least 50% of its initial activity after one PCR cycle. "Active in salt concentration" means an enzyme which shows preferably at least 5% and better at least 20% and still better at least 50% of its activity compared to the activity in solution with salt being lower than 25 mM.

In a preferred embodiment, the amplification chamber 1 is sealed to allow liquid evaporation by less than 10 %, preferably by less than 1% of the solution present in the amplification chamber 1 after 35 amplification cycles.

In another embodiment, the reagent (possibly present in the reagent chamber 2) allows to perform an amplification independently from the amplification performed in the first chamber 1.

In another preferred embodiment, the reagent (possibly present in the reagent chamber 2) is a reagent for fragmenting amplified target nucleotide sequences contained in the amplification chamber 1. This step is used to reduce the length of the amplified target nucleotide sequences (amplicons) and thus favour the hybridization of the possibly labelled fragments on capture probes of the micro-array. Preferably, the reagent for fragmenting the amplified target nucleotide sequence into fragments is selected from the group consisting of: DNase, alkaline solution and metal ions.

In the specific embodiment including the fragmenting step of the amplified target nucleotide sequences (amplicons) and the treatment of the amplified target nucleotide sequences (step 2) are performed in a chamber different from the detection chamber 3 containing the micro-array 16. The flowchart of these steps performed in the method of the invention with the device of the invention is illustrated in figure 2 and schematic representation of the device in figures 3-5.

In this specific embodiment, the device further comprises:
- a fragmentation chamber 4 to cut (for a fragmentation of) amplified nucleotide sequences into fragments, the said fragmentation chamber being connected to the amplification chamber 1 and to the reagent chamber 2 by the first and second outlet channels 13, 14 and being connected to the detection chamber 3 by a third outlet channel 19 and wherein said first and second channels 13, 14 are located at a different level than the third channel 19.

In still another embodiment, the device further comprises one or more additional chamber(s) connected to one or two of the other chambers 1,2,3,4 for performing one or more additional steps such as purification, labelling of the fragments or the amplified target nucleotide sequences.

In a preferred embodiment, the device further comprises at least one adhesive, cap 29 or any other element for closing the opening of the first and/or second inlet port(s) 11,12. The adhesive is used to hermetically close the device once the consumer has introduced solutions in the amplification chamber 1 and in the reagent chamber 2.
In a specific embodiment, the detection chamber 3 is composed of two broad flat surfaces with one surface bearing the capture probes specific of the amplified target nucleotide sequences (amplicons which are amplified in the PCR) and one surface has a thermo-conductance at least 2 times and even 4 times higher than the other one. In a particular embodiment, the surface having the higher thermo-conductance is thinner than the other surface by at least a factor of 2 and better a factor of 4.
In still another embodiment, these chambers are considered as separated vessels.

The invention also relates to an automate for a detection of a nucleotide sequence of an organism, said automate comprising:
- the device 10 of the invention,
- a temperature control system adapted to perform an automated amplification process of the nucleotide sequence in the first (amplification) chamber 1 and to perform an hybridization of the amplified target nucleotide sequence(s) in the third (detection) chamber 3 of the device 10,
- one or more mean(s) to transfer a solution from the first (amplification) 1 and the second (reagent) chambers 2 of the device to the third (detection) chamber 3 or to the fourth (fragmentation) chamber 4,
- a detector 43 for reading a signal resulting from a binding between the amplified target nucleotide sequence(s) and their corresponding capture nucleotide molecules(s) fixed upon the solid support surface 15 of the detection chamber 3.

In case the solution has been transferred to the detection chamber 3 of the device, the transfer mean allows a solution transfer from detection chamber 3 back to the amplification chamber 1 and/or to the reagent chamber 2.

In case the solution has been transferred to the fragmentation chamber 4 of the device, the transfer means allows a solution transfer from the fragmentation chamber 4 to the detection chamber 3.

In a related embodiment, the transfer mean allows a solution transfer from this detection chamber 3 back to fragmentation chamber 4.

In a preferred embodiment, the solution transfer mean (from the first (amplification) chamber 1 and second (reagent) chamber 2 to the third (detection) chamber 3 or to the fourth (fragmentation) chamber 4) is a centrifugation force.

In another embodiment, the solution transfer mean (from the first (amplification) chamber 1 and second (reagent) chamber 2 to the third (detection) chamber 3 or to the fourth (fragmentation) chamber 4) is a pressure force.

In a further embodiment, the solution transfer mean (from the first (amplification) chamber 1 and the second (reagent) chamber 2 to the third (detection) chamber 3 or to the fourth 4 chamber) is a mechanical movement.

In another embodiment, the solution transfer (from the first (amplification) chamber 1 and the second (reagent) chamber 2 to the third (detection) Chamber 3 or to the fourth (fragmentation) chamber 4) is an electric device.

In a preferred embodiment, the reading detector 43 of the signal resulting from the binding between amplified nucleotide sequences and capture nucleotide molecule is performed in presence or absence of the solution containing the amplified nucleotide sequences.

In a specific embodiment, the reading detector 43 of the signal resulting from the binding between amplified nucleotide sequences and capture nucleotide molecule(s) comprises an evanescent field detector. Preferably the evanescent field detector generates an incident light source 41 that illuminates a side 40 of the device 10.

In a preferred embodiment, the incident light source 41 is a non collimated laser source or a light emitting diode by a pair of optical fibber bundles.

In another embodiment, the side 40 of the device forms an angle comprised between about 90° and 110° with solid support surface 15 of the device 10.

In an alternative embodiment, the evanescent field detector generates an incident light source 41 that illuminates the solid support surface 15 of the device 10 with an incidence angle comprised between about 10° and 90°.

In a preferred embodiment, the evanescent field excites a label of the amplified nucleotide sequences and the emitted signal is detected by the reading detector comprising preferably a CCD camera.

In another embodiment, the incident light source 41, the device 10 and the detector 43 are not moving relative to each other.

The temperature control system comprises an active temperature control system and a temperature control unit, allowing to regulate precisely the temperature and to perform temperature cycles.

The active temperature control system may be a Peltier element, a micro-thin wire heating element laid in a pattern between optical grade polyester sheets like Thermal-Clear™ transparent heaters from Minco, or fluidic system circulating externally temperature regulated fluid.

In a preferred embodiment, the temperature control system is a PCR thermocycler.

In another preferred embodiment, the temperature control system comprises a Peltier element.

In another embodiment, the temperature control system comprises a thermoblock 32 of 96 wells which further comprises a thermic adaptor 30 which is placed between the device 10 and the thermoblock 32 and which is adapted to fit with the device 10 and the thermoblock 32.

In another preferred embodiment, the temperature control system comprises a thermoblock 32 made of at least four segments 33, each segment 33 being adapted to receive a device 10 and wherein each segment 33 comprises a flat surface 34 and two cavities 35 adapted to receive the amplification chamber 1 and the reagent chamber 2 of the device 10. A preferred thermoblock is illustrated in figure 11.

The device of the invention is particularly adapted for a detection (identification) and/or quantification method of multiple single nucleotide polymorphisms or multiple mutations (multiple SNPs) present at different gene locus. Preferably, said detection or characterization method is obtained upon the same micro-array present in the device of the invention.

In preferred embodiment, the nucleotide sequence to be detected in the device of the invention (with the method of the invention) is a nucleotide base or SNP.

The device is preferably positioned at an angle of 80° relative to the direction of the applied centrifugal force (full arrow). The hybridization chamber (3) is located far from the center of the rotor while the fragmentation chamber (4) is located close to the center of the rotor. Rotation facilitates the transfer of liquid from the fragmentation chamber to the hybridization chamber.

The genetic amplification step used in the method with the device of the invention is performed by amplification protocols well known in the art, preferably by a method selected from the group consisting of PCR, RT-PCR, LCR, CPT, NASBA, ICR or Avalanche DNA techniques.

In a preferred embodiment, the nucleotide sequence amplification is a PCR. In the first step of the method of the invention, the nucleotide sequence of a gene is amplified using at least one primer pair (i.e. a pair of two different primers).

However, several primer pairs are either used for amplifying the different specific nucleotide sequences of a gene, these sequences being preferably different exons, or used for amplifying different genes or different parts of a cell genome.

Preferably, each amplified target nucleotide sequence comprises several loci. All these loci of the target are then amplified with the same primer pair being consensus primers for an amplification of all these loci, but each locus is detected on specific capture probes.

Therefore, the micro-array contains capture probes specific for one or more loci for hybridization with amplified target nucleotide sequence(s) comprising the SNP to be detected in each locus, the different mutated bases being located in the same exon or in different exons originating from the same gene or from different genes, preferably present in the same nucleotide sequence. The amplification step of these several exons is preferably obtained with different primer pairs, each primer pair being specific for one exon. Amplification of several exons is preferably performed in the same conditions for all exons in the PCR chamber 1.

Parts (or portions) of the gene or genome sequence (loci) having possible mutations to be detected can be firstly amplified by PCR and the resulting amplified target nucleotide sequences (amplicons) are fragmented by DNAse treatment in chamber 4 (Grimm et al. 2004, Journal of Clinical Microbioloy, 42, 3766-3774). In the preferred embodiment, the resulting amplicon fragments are between 30 and 70 bases long. The distribution of the fragments size obtained after fragmentation of the amplicons (into fragments) is advantageously checked by analysis by capillary electrophoresis (Bioanalyser, Agilent) and the average size distribution of the pieces is preferably comprised between 30 and 70 bases long.

In a preferred embodiment, the capture nucleotide probes (molecule) comprises a spacer portion (linker) and a capture portion (specific part of the capture nucleotide probes complementary to the amplified nucleotide sequence) being capable to bind by complementary base pairing with amplified nucleotide sequences or their fragments.

In another embodiment, the capture portion is preferably comprised between about 10 and about 100 bases, preferably between about 15 and about 40 bases and more preferably between about 18 and about 24 bases. These bases are preferably assigned as a continuous sequence located at or near the extremity of the capture probes (nucleotide sequences). This sequence is considered as a specific sequence for the detection of the amplified target nucleotide sequence(s) or their fragments.

In a preferred embodiment, the capture nucleotide probes comprises:
- a capture portion of 10 to 100 nucleotides that is capable to bind by complementary base pairing with a specific sequence of the amplified target nucleotide sequences (or their fragments) such that said capture portion defines two non-complementary ends of these amplified target nucleotides sequences (or their fragments) and,
- a spacer portion having at least 20 nucleotides, and wherein the two non-complementary ends of the amplified target nucleotide sequences comprise a spacer end and a non-spacer end, such that the spacer end is non-complementary to the spacer portion of the capture nucleotide probe, and said spacer end exceeds said non-spacer end by at least 50 bases.

In a preferred embodiment, the spacer portion is a polynucleotide being at least about 20 nucleotides long, at least about 50 or about 70 nucleotides and preferably at least about 90 nucleotides long. The spacer portion is a given nucleotide sequence being homologous to none of the genome sequence (when using an identity of at least about 10 and better about 5 consecutive bases). To avoid non specific hybridization, there will be no more than around about 15 consecutive complementary base pair bindings between a target polynucleotide (or nucleotide) sequence and the spacer portion, preferably there will be less than about 10 such pairings possible, more preferably less than about 5. As such, the nucleotide sequence of the spacer portion will contain, preferably less than about 15 bases and more preferably, less than about 10 and still more preferably less than about 5 contiguous bases complementary to the target nucleotide sequences to be detected. The determination of possible consecutive sequences is easily done by comparison of the sequences to molecular database as provided by Genbank and using software such as nucleotide-nucleotide BLAST(blastn) :
(http://www.ncbi.nlm.nih.gov/BLAST)
The spacer portion is preferably located at the 5' extremity of the capture nucleotide molecule being fixed to the surface of the solid support by a covalent link present at the 5' end or nearby. The capture portion is preferably located at 3' end of the capture nucleotide probe (free extremity not bound to the support) at 1 to 23 nucleotides from the end.

The total length of the capture probes (nucleotide sequences) including the spacer portion is comprised between about 30 and about 300 bases, preferably between about 35 and about 200 bases, more preferably between about 39 and about 120 bases. In another preferred embodiment of the invention, capture probes (nucleotide sequences) are chemically synthesised oligonucleotide sequences of about 100 bases, which may e.g. be easily performed on programmed automatic synthesizer. Such sequences can bear a functionalized group for covalent attachment upon the support, at high concentrations. Longer capture probes (nucleotide molecules) are preferably synthesised by PCR amplification of a sequence incorporated into a plasmid containing the capture portion of the capture nucleotide probe (molecule) and spacer portion.

In a preferred embodiment, multiple capture nucleotide probe (molecules) are fixed in the form of a micro-array having at least 4 different discrete regions.
On the micro-array, capture probes are arranged at predetermined locations at a density of at least about 4, about 10, about 16, about 20, about 50, about 100, about 1000, about 4000, about 10 000 or more, different capture probes/cm² insoluble solid support surface. The capture probes are advantageously covalently attached to the surface of the solid support (preferably a non porous solid support surface) by one of their extremities, preferably by their 5' end. The sensitivity may be further increased by spotting capture probes on the solid support surface by a robot at high density according to an micro-array. The amount of capture probes spotted on the micro-array is preferably comprised between about 0.01 to about 5 picomoles of sequence equivalent/cm² of solid support surface.

The capture probes preferably differ by one base (SNP) located at about 4 to about 10 and preferably at about 4 to about 6 bases from the (free) 3' end of the target specific part (portion) of the bound capture probe. The array may contain specific capture nucleotide probes (molecules) for each base of a specific locus to be detected. The bases to be detected are present within one or several exons of the same gene nucleotide sequence or from different gene nucleotide sequences.

In a preferred example, the array contains specific capture probes for the detection of SNP in human Cytochromes P450 2C9, 2C19 and 2D6. The array may contain specific capture probes for the detection of several SNP in one gene nucleotide sequence, or the array may contain specific capture probes for the detection of several SNP in different gene nucleotide sequences. The amplified target nucleotide sequences are labelled during the amplification step. The labelled associated detections are numerous. A review of the different labelling molecules is given in W0 97/27317. They are obtained using either already labelled primer or by incorporation of labelled nucleotides during the amplification step or fragmentation step.

The preferred labels are fluorochromes which are detected with high sensitivity with fluorescent detector. Fluorochromes include but are not limited tocyanin dyes (Cy3, Cy5 and Cy7) suitable for analyzing an array by using commercially available array scanners (as available from, for example, General Scanning, Genetic Microsystem). Preferably, the excitation wavelength for cyanin 3 is comprised between 540 and 558 nm with a peak at 550 nm, and the emission wavelength is comprised between 562 and 580 nm with a peak at 570 nm.

Preferably, the excitation wavelength for cyanin 5 is comprised between 639 and 659 nm with a peak at 649 nm, and the emission wavelength is comprised between 665 and 685 nm with a peak at 670 nm. Preferably, the excitation wavelength for cyanin 7 is comprised between 733 and 753 nm with a peak at 743 nm, and the emission wavelength is comprised between 757 and 777 nm with a peak at 767 nm.

In a preferred embodiment, the detection of the fluorescence signal related to the presence of the amplified target nucleotide sequences (amplicons)or their fragments on the capture probe (molecule) is performed in absence of solution containing the labelled amplified target nucleotide sequences(amplicons)or their fragments. This is easily obtained by flipping the device in order to transfer the content of the detection (hybridization) chamber 3 back to fragmentation chamber 4 or to the PCR chamber 1.

In another embodiment, the detection of the fluorescence signal related to the presence of the amplified target nucleotide sequences (amplicons)or this fragments on the capture molecule is performed in presence of the solution containing the labelled amplified target nucleotide sequences (amplicons)or their fragments. Preferably, the detection takes party of a signal increase on the micro-array as compared to the fluorescence in solution.

In a particular embodiment, the difference of the detection of the fluorochrome present on the array is based on the difference in the anisotropy of the fluorochrome being associated with a bound molecule hybridized on the capture probe as a DNA double helix compared to the free moving molecule in solution. The anisotropy depends on the mobility and the lifetime of the fluorochromes to the detected. The method of assay for the anisotropy on array is now available from Blueshift Biotechnologies Inc., 238 East Caribbean Drive, Sunnyvale, CA 94089 (http://www.blueshiftbiotech.com/dynamicfl.html).

In a particular embodiment, the detection of fluorophore molecule is obtained preferably in a time-resolved manner. Fluorescent molecules have a fluorescent lifetime associated with the emission process. Typically lifetimes for small fluorophore such as fluorescein and rhodamine are in the 2-10 nanosecond range. Time-resolved fluorescence (TRF) assays use a long-lived (>1000 ns) fluorophores to discriminate assay signal from short-lived interference such as autofluorescence of the matrix or fluorescent samples which almost always have lifetimes much less than 10 ns. Lifetime is preferably modulated by the presence in the vicinity of another fluorophore or a quencher with which a resonant energy transfer occurs. Instruments for TRF simply delay the measurement of the emission until after the short-lived fluorescence has died out and the long-lived reporter fluorescence still persists. Fluorescence lifetime can be determined in two fundamental ways. The time domain technique uses very short pulses (pico-seconds) of excitation and then monitors the emission in real time over the nanosecond lifetime. Fitting the decay curve to an exponential yields the lifetime. The frequency domain technique modulates the excitation at megahertz frequencies and then watches the emission intensity fluctuate in response. The phase delay and amplitude modulation can then be used to determine lifetime. The frequency technique for fast and economical lifetime imaging is now available from Blueshift Biotechnologies Inc.

In a preferred embodiment of the invention, the step of detecting the hybridized amplified target nucleotide sequences (amplicons)or their fragments takes party of a fluorescence signal of the amplified target nucleotide sequences (amplicons) or their fragments lower in solution than on the hybridized capture probe.

In a particular embodiment, the lower fluorescent signal of the amplified target nucleotide sequences (amplicons) or their fragments in solution compared to the hybridized amplified target nucleotide sequences (amplicons) or their fragments is obtained by quenching of the fluorochrome. A primer is labeled with a fluorochrome which is fluorescent when free in the solution and is quenched when incorporated into the amplified target nucleotide sequences (amplicons) or their fragments. The fluorescence quenching is preferably obtained by using a quencher such but not limited to Dabcyl incorporated in the second non fluorescent (amplicon) strand. One specific embodiment used the Plexor^{™} Technology (Promega). This technology takes advantage of the highly specific interaction between two modified nucleotides: isoguanine (iso-dG) and 5'-methylisocytosine (iso-dC). In the real time PCR reaction, one primer is synthesized with an iso-dC residue and a fluorochrome at the 5'end. The second primer is unlabeled. Iso-dGTP nucleotides, modified to include Dabcyl as a quencher, are included in the reaction mix. During the amplification only Dabcyl-iso-dGTP is incorporated at the position complementary to the iso-dC residue and as a result of the close proximity between the two residues, the fluorescence is quenched. The hybridization of the amplified target nucleotide sequence (amplicon) or their fragments strand carrying the fluorochrome on the capture probe would restore the fluorescence emission.

In an alternative embodiment, the lower signal of the amplicons or their fragments in solution is obtained by a difference in the optimal wavelength of fluorescence excitation between the amplicons present in solution and immobilized on the capture molecule. In still another embodiment, the lower signal of the amplicons in solution is obtained by a difference in the optimal wavelength of fluorescence emission between the amplicons present in solution and immobilized on the capture probe.

Preferably, the difference in the wavelength of fluorescence emission is obtained by fluorescence resonance energy transfer (FRET). In one specific embodiment, a primer is labeled with a fluorochrome (F1) having a given optimal fluorescent emission wavelength and serving as donor that is fluorescent when excited at its excitation wavelength in the solution. The incorporation of the primer into the amplicon at proximity of a fluorochrome acceptor (F2) would result in an optimal fluorescence emission wavelength different from the fluorochrome F1. By detecting the fluorescence emission at the wavelength corresponding to the optimal emission of F1, the signal will be optimal for the hybridized amplicons and will be lower for the amplicons present in the solution. Particularly, the primer is synthesized with an iso-dC residue and a fluorochrome donor (i.e. TAMRA) at the 5'end and the solution contains Iso-dGTP nucleotides, modified to include a fluorochrome acceptor (i.e. Cy5). During the PCR, the amplicons are formed with the two fluorochromes being at close proximity as explained previously for the Plexor^{™} Technology (Promega). Detection is then performed using an excitation/emission wavelength optimal for the donor. As a result of the close proximity between the donor and the acceptor, the detected fluorescence is decreased in solution. The hybridization of the amplicon strand carrying the donor on the capture molecule would restore the optimal fluorescence emission.

The above described methods allow a better discrimination between the amplified target nucleotide sequences (amplicons/targets) present in solution and hybridized on the capture probes.

In a preferred embodiment, the excitation of the fluorophore molecule is obtained preferably on the fluorophore present on target bound to the capture probe rather that on the fluorophore present in the solution. Homogeneous detection of the bound targets (amplified target nucleotide sequences or their fragments) is critical especially when the capture probes are immobilized on the solid support in the form of an array which occupies a certain surface on the solid support. Excitation of the bound targets should be highly uniform and/or constant whatever their position on the solid support. Homogeneous excitation is best obtained through homogeneous illumination. Homogeneous excitation can be obtained, for example, using the apparatus disclosed herein.

In a preferred method, the excitation is obtained by a laser beam which is focussed on the surface of the array. Scanner method with a focusing of the laser beam used a confocal scanning method including a pin hole. Many such scanners are commercially available such as the PROSCANARRAY® line of scanners from PerkinElmer® Life, the Affymetrix 428 scanner, the Virtek Vision Chipreader line, etc. Some fluorescence laser based detection is now available for multiwell formats as for example the Safir from Tecan (Tecan Trading AG, Männedorf, Switzerland; www.tecan.com). They could be adapted for the present invention.

Illumination is such as to obtain total internal reflection fluorescence (TIRF) and homogeneous evanescent field on the surface of the solid support having capture probes immobilized thereon. The person skilled in the art may use the following described method with the device or apparatus of the invention.

US Patent No. 5,750,337 (which is hereby incorporated by reference herein) relates to a method using TIRF to measure hybridization of analyte DNA with a probe that is associated with an evanescent wave detector waveguide. An intercalating fluorescent dye is used to label specifically the obtained double stranded DNA. For an efficient detection (and/or quantification), the person skilled in the art may refer to known techniques described in the state of the art.

US Patent publication 2004/0091862 (which is hereby incorporated by reference herein) relates to a method for determining association/dissociation of complexes made of at least two components by TIRF. The first component, which is in a liquid phase, is contacted with second component linked to a solid reaction carrier comprising a planar optical waveguide. The excitation of fluorescent dyes bound to the components located close to the surface is transmitted by TIRF so that fluorescent light is emitted. A publication of the same author (Lehr et al. Sensors and Actuators B 92 (2003), 303-314) relates to a model system for the detection of hybridization events of single strand oligonucleotides on micro-arrays in real time by employing the principle of TIRF. Three TIRF-sensing configurations were compared, which comprise a light source, an optical system and a detector. The model-array comprised a substrate of printed Cy5 molecules thereon and a solution containing Cy5 molecules.

In a preferred embodiment, the excitation of the fluorochrome is obtained by illumination the sides of the array substrate so as to give the excitation to the molecules close to the surface.

In a preferred embodiment, the device for detecting a signal comprises a light source illuminating the sides of the insoluble solid support. The light source is preferably a diverging laser beam or a light emitting diode. Preferably, the light beam is transmitted through a pair of optical fibber bundles as proposed by Aurora Photonics Inc. (26791 West Lakeview, Lake Barrington, USA; info@auroraphotonics.com) and as disclosed in US Patent No. 6,620,623 (which is hereby incorporated by reference herein). The non collimated laser source is preferably a low power non collimated laser diode or a light emitting diode (LED). Preferably, the power ranges between about 3 mW and about 5 mW. The non collimated laser diode emits at a specific wavelength, preferably between about 470 nm and about 650 nm. Alternatively, a LED coupled with optical filter can also be used as an illumination source. A pair of fibber optic bundles directs the light to opposing sides of a glass substrate. The glass substrate or slide supports a bio-array. The light is directed to the opposing sides of the glass substrate by the fibber optic bundles that are, for example, formed by borosilicate fibber light guides, quartz fibber light guides or plastic fibber light guides or fibber light guides formed by another suitable material. The fibber optic bundles are carried by a positioner and are splayed out to make a respective fibber optic fan. The fibber optic fans are one fibber thick, each defining a light line or linear array of a plurality of optical fibbers. Each of the fibber optic bundles includes a plurality of optical fibbers providing generally symmetrical illumination to the opposing sides of the glass substrate. Each optical fibber includes a polished face positioned proximate to a divergent diffuser. The polished optical fibber faces defining the light line transfer laser light to opposing sides of the glass substrate via the divergent diffuser with only a small percentage of the laser light going back into the optical fibber. This illumination process of the preferred embodiment randomizes any non-uniformity in the laser source, creating a more uniform illumination source.

In still another embodiment the fluorescence excitation is provided through fibber optics on which the capture molecules are fixed and the person skilled in the art may use the means and methods described in the following documents.

US Patent No. 6,503,711 (which is hereby incorporated by reference herein) provides a system based on the use of an index of refraction of the immobilized layer equal to or greater than the refractive index of the interaction surface of the optical element such that direct excitation of the fluorophore in the immobilization layer results in the detection of the target nucleic acid. US Patent No. 5,494,798 (which is hereby incorporated by reference herein) relates to a similar fibber optic sensor for immunoassay.

Some fluorescent labels may be of particular interest, such as nanocrystalline particles having fluorescent properties. The most common ones are the Quantum dots (Han et al., Nature Biotechnology, Vol. 19, p.631, 2001). They are fluorescent and do not bleach with time or with illumination. Their stability makes them particularly suitable for the use in continuous reading, as proposed in this invention. Also, they contain metals that confer to these particles specific properties, so that other methods than fluorescence can be used to monitor their attachment to the capture probes.

Thermal heating of these particles is one of the parameters that may be monitored with time. The fact that the metal absorbs the energy of a light beam, preferably a laser beam, and induces heating of the particle, has been used as a basis for the detection of low density gold particles on a support, and even single particles are detected (Boyer et al., Science, Vol. 297, p.1160, 2002). The method is called Photothermal Interference contrast.

Direct method for a detection and/or a quantification of the binding of the target molecules on capture probes of the micro-array is the chemical cartography based on optical process of non-linear generation frequency spectroscopy (GFS) (L. Dreesen et al., Chem Phys Chem, Vol.5 , p.1719, 2004).

This technology allows the imaging in real time of the vibrational properties of surfaces and interfaces with a submicron spatial resolution. The measurement is obtained by mixing at the surface of a substrate two laser beams, one having a fixed frequency in the visible (green) and the other having a variable frequency in infrared. The vibrational signature at the interface is obtained by measuring the light emitted by the sample in function of the frequency of the infrared laser beam. This method avoids labeling the target to be detected and so it represents a particular embodiment of the invention.

Another technology for the direct measurement of nanoparticles is Rayleigh scattering. This method is based on the use of a light beam adapted in order to obtain an oscillation of the electrons in a metal particle so that an electromagnetic radiation is obtain from the particle, which can be detected. (Stimpson et al., Proc. Natl. Acad. Sci. USA, Vol. 100, p.11350, 2003) (real-time detection of DNA hybridization and melting on oligonucleotide arrays by using optical wave guides) However until now the method is lacking the necessary sensitivity for application on biological samples.

Alternatively, Raman scattering and surface plasmon resonance may be applied in the present invention, which techniques have been extensively used for the detection of antibody/antigen binding, but are also well suited for the multiparametric measurement of the arrays and for the required sensitivity on biological samples. (Thiel et al., Analytical Chemistry, Vol.69, p. 4948, 1997).

In another embodiment, quartz crystal microbalances may be applied, which are now sensitive enough that they can measure changes of mass less than one nanogram (cf. Caruso et al., Analytical Chemistry, Vol. 69, p.2043, 1997). This is one proposal for micro-array detection in real-time.

Cantilevers are another option for the detection of DNA on micro-arrays. (McKendry et al., Proc. Natl. Acad. Sci. USA, Vol.99, p.9783, 2002).
Also, another technology is the electrical detection of nanoparticles, which takes into account their metal properties. Electrochemical detection was first applied, but with low sensitivity. A more advanced and more sensitive method is the detection by differential pulse voltametry (Ozsoz et al., Analytical Chemistry, Vol. 75, p.2181, 2003).

The electrical resistance and the capacitance properties of the metal are among the best properties to be detected on electronic chips. The presence of a metal between two electrodes induces a change in the electric properties of the chips or the electrodes including change of electrical resistance or conductance and/or of capacitance and/or impedance. The detection of the DNA or proteins is then observed when the capture probes are present on one of the electrodes (Moreno-Hagelsieb et al., Sensors and Actuators B-Chemical, Vol. 98, p.269, 2004). The capacitance assay of gold labeled DNA has been described by Guiducci et al. (Biosens Bioelectron, Vol.19, p.781, 2004). Since electronic chips can be made to comprise several plots, different targets may be detected on different plots and the change in the electrical resistance or in the capacitance may be recorded. One promising method is the use of interdigitated electrodes which allows compatibility between the array pattern and the location on the electrodes.

Although these methods have not yet been able to produce the reliable and sensitive detections required by biological samples, some of them will succeed to fulfil the requirements for real-time detection (see review of the detection methods for the nanoparticles by Foultier et al., IEE Proc. Nanobiotechnol., Vol. 152, p.3, 2005).

Another method for the detection of the particles is to count them according to their location on the array by optical method such as described by Blab et al. (Biophysical J., Vol.90, p.L13, 2006). The method relies on Laser Induced Scattering around a NanoAbsorder (LISNA). It provides direct counting of individual nanoparticles on each spot of the array.
Preferably, the detection of the label is performed in a detector selected from the method group consisting of : colorimetry, fluorescence, time-resolved fluorescence, photothermal interference contrast, Rayleigh scattering, Raman scattering, surface plasmon resonance, change of mass, quartz crystal microbalances, cantilevers, differential pulse voltametry, chemical cartography by non linear generation frequency spectroscopy, optical change, resistivity, capacitance, anisotropy, refractive index and/or counting nanoparticles.

Preferably, the fluorescent scanner uses a laser beam including a confocal scanning method and also preferably a pin hole. A light source generates a beam of light to excite the labeled targets on the support. The light source may be a laser that generates a beam having a wavelength of about 532 nm delivered at a power of about 15 mW with a divergence that may be below 1.2 mrad.

The laser beam generated by laser is preferably nearly collimated and nearly Gaussian. An exchangeable excitation filter may be used to collect only the wavelengths of interest. An additional filter wheel may be placed and be used as an attenuation filter to regulate precisely the laser power. This filter wheel may be shaded differently at variable know absorption levels. A lens that may be anti-reflection coated can be used for focusing the laser beam on the micro-array. The distance between the light source, the lens and the support may be variable to allow focusing.

Thereafter, the light passes through a dichroic mirror. This mirror may pass light having a wavelength lower than about 530 nm, but reflect light having a wavelength greater than 560 nm. Consequently, the 532 nm light coming from the laser is passed through the dichroic mirror to the support. The light then passes through third chamber and reaches the micro-array, where bound labeled target are excited and emit fluorescence at about 560nm. Emitted fluorescence is reflected on the dichroic mirror since its wavelength is greater than about 560 nm to a microscope objective for magnification of the image sample. The fluoresced light is then focused to a photomultiplier tube for detecting the number of photons present therein. In a specific embodiment, an additional emission filter that transmits light having a wavelength greater than about 550 nm may be added. Thus, photomultiplier tube detects substantially only fluoresced light. The Photomultiplier tube generates a pulse for each photon detected. Each of these pulses is amplified and converted to an electronic signal by photoelectric effect. A data acquisition board then collects the resulting signals.

In a preferred embodiment, the device is fixed during the signal detection and the optical system moves relative to the device to scan the micro-array.

In another embodiment, the device moves relative to the optical system to scan the micro-array. After data are collected from a region of the micro-array, the device moves so that light can be directed at a different discrete region of the micro-array. The process is repeated until all discrete regions of the micro-array have been scanned.

In still another embodiment, the resolution of the optical system is between 0.1 microns and 500 microns and more preferably between 10 and 100 microns.

In a preferred embodiment, the device is made of a solid material which is resistant to 90°C preferably to 95°C. Particularly, the amplification chamber of the device has to resist to the temperature of the amplification process, preferably the temperature cycles of a PCR. The device of the invention may also comprises an inlet for the fragmentation chamber (4) for the introduction of the DNase that can be present before the assay in a lyophilised medium in this fragmentation chamber. Alternatively, one may introduce the DNase in this chamber with the addition of a washing buffer before the transfer of one chamber to another. The fragmentation chamber 4 (where the fragmentation is performed) has to be heated to 95°C to inactivate the DNase after the fragmentation. The detection chamber (where the hybridization is performed) has to be heated at 45-70°C to perform the hybridization.

In a particular embodiment, the device comprises or is made of a solid material selected from the group consisting of glass, a polymer material, or a mixture thereof. The polymer material is preferably selected from the group consisting of: polycarbonate (PC), polyethylene (PE), Cycloolefin copolymer (COC), cyclicolefin polymer (COP) or a mixture thereof. In still a specific embodiment, cycloolefin polymer is preferably ZEONEX® 330R or ZEONOR® (Zeon Chemicals, Louisville, USA), Topas, Udel, Radel or THV.

The preferred device for detection of nucleotide sequences is made with 3 different parts as shown in figure 5. The constitutive polymer of the different parts is cycloolefin copolymer (COC) Zeonex 330R. This polymer presents excellent optical properties, chemical resistance, thermal stability at 95°C, low fluorescence. The same polymer is used for the three parts to allow an optimal welding between the different parts.

The three different parts (see figure 5) are the following:
the first Part (A) is a transparent Zeonex slide (500 µm thick) holding two holes fitting with top of two chambers (1, 2) and two small holes at two corners for positioning onto the upper side of a second part . The aim of this part A is to seal the upper side of the second part B.
the second Part (B) is made of black Zeonex plastic and is the essential part of the device welded to the first and third parts (A,C). It is made of two sides (upper B and lower B') carrying elements which are in different plans.
   The upper side B of the second part comprises two chambers (1, 2), each chamber been connected to a channel. The two channels connect together to make one channel crossing the whole device to reach the fragmentation chamber 4. The fragmentation chamber 4 is 100µl in volume and is connected to a hybridization (detection) chamber 3 being located in another plan (lower side B'). The dimension of the hybridization (detection) chamber 3 is 2 x 2 cm and 330 µm thick.
The third Part (C) is a transparent Zeonex slide (500 µm thick) holding two holes for positioning onto the lower side of the second part (B'). The aim of this third part C is to seal the fragmentation chamber 4 and the hybridization (detection) chamber 32.
   The two chambers 1, 2 have to be closed by tube caps or a sealing film before performing a microfluidic reaction.

In one embodiment, the device 10 is inserted an automatic machine controlling liquid solution injection into the chambers 1 and 2 and their sealing as being developed in the microfluidic technology.

### Example 1. End point measurement of a PCR product in the device

### 1. Preparation of Zeonex third Part of the device activated with aldehyde groups.

The third Part C of the device (figure 5) was made of transparent Zeonex 330R. The dimensions of third Part C are the following: 0.8 mm width, 1.5 mm long and 1 mm thick. The third Part C was modified with addition of specific chemical and reactive groups such as aldehyde groups according to the process disclosed in application EP 06112775.9.

### 2. Capture probe immobilization on the third Part C of the device functionalized with aldehydes

Aminated capture probe were then spotted in a micro-array pattern on third Part C of the device 10 comprising aldehyde groups. The aminated capture probes were spotted from solutions at concentrations of 3 µM. After the spotting, the third Part C was washed once for one minute with 0.2% SDS, twice with distilled water. The third Part C was then incubated for five minutes with NaBH₄ solution (2.5 mg/ml of PBS 75%/ Ethanol 25%), washed twice with distilled water, then once at 100°C for five minutes and dried. The coverslips were stored under vacuum at 4°C.

The capture portion of the capture molecules used in this experiment had the following sequences:
TP35S (SEQ ID NO: 1): 5'- GTCATCCCTTACGTCAGTGGAGATAT -3'
TPAT1 (SEQ ID NO: 2): 5'- CTGTGTATCCCAAAGCCTCATGCAA -3'
Each capture probe comprised a spacer portion of 90 bases long at the 5' end of the capture portion, said spacer having the following sequence:

5'Amine-
AAAGTTGAGTCCATTTGTGATGCTAGAAAAGTTGGAACTTTCTTGAACGTCTCCTA
TATGTCATACATGAATAGGTTGATTTTACTGTAC-3' (SEQ ID NO: 4)

### 3. Preparation of the device

The first Plastic Part A (transparent Zeonex) and the second Part (B,B') (black Zeonex) of the device 10 were welded together using a Leister Micolas (Leister Process Technology, Sarnen, Switzerland) with a diode laser 808 nm to form two PCR tubes, one fragmentation chamber 4 and one hybridization (detection) chamber 3 as shown on figure 5. Then the two Parts B and C) (surface carrying a micro-array obtained in step 2) were welded together so that the fragmentation chamber 4 and the hybridization (detection) chamber 3 were completely closed and the micro-array 16 was located inside the hybridization (detection) chamber 3.

### 4. PCR and detection

PCR was designed for the amplification of the 35 promoter element of DNA sample of a genetically modified organism (GMO) Bt11 from reference flour ERM-BF412f. The primers used in this experiment have the following sequences:
OP35SF (SEQ ID NO: 5): 5'- Cy3- CGTCTTCAAAGCAAGTGGATTG -3'
OP35SR (SEQ ID NO: 6): 5'- TCTTGCGAAGGATAGTGGGATT -3'

One positive hybridization control was added to the reaction mixture which is complementary to the capture probe TEPSPS1 (SEQ ID NO: 3). This control has the following sequence:

EPSPS1 (hybridization control) (SEQ ID NO: 7): 5′- Cy3-
TCGGACAGGGCGGCGAGTAGGAG -3′

The amplified target nucleotide sequence had part of one of its strand sequence specific of capture portion of the probes P35S (SEQ ID NO: 1).
A mix for PCR reaction was prepared as followed: for a final volume of 100 µl, mix ten µl of PCR Eppendorf buffer, ten µl of dNTP mix (each of dNTP at a final concentration of 200 µM), one µl of twenty µM primer OP35SF-Cy3 labelled at 5' end and 0.5 µl of twenty µM primer OP35SR, two µl of Eppendorf Taq DNA polymerase, 68.5 µl of water and eight µl of twenty ng/µl of DNA sample extracted from reference flour ERM-BF412f.

One PCR tube of the device was then filled with 100 µl of PCR reaction mix. The second PCR tube was filled with hybridization mix containing Phosphate buffer 0.6 M pH 7.4 and 500 nM EPSPS1 (SEQ ID NO: 7) labeled with Cy3. The PCR tubes (chambers 1 and 2) were closed with an Eppendorf PCR sealing film. The PCR tubes (chambers 1 and 2) of the device 10 were positioned in a thermic adapter 30 (as shown in figure 10) which is then inserted into a PCR thermocycler (Eppendorf, Germany). The thermocycler was programmed as followed: 95°C (denaturation) for 30 sec then going to 56°C (annealing) for 30 sec and then for 30 sec at 72°C (elongation). The same cycle is repeated 34 times.

Then the closed device 10 was positioned horizontally onto the centrifugation adaptor (21) of the centrifugation rotor 20 (as shown in figure 8) and centrifuged for 15 sec at 1000 rpm to allow liquid to go from PCR tubes (chambers 1 and 2) to the fragmentation chamber 4 where the content of the two PCR tubes were mixed together.

The device was then switched in the centrifuge and positioned at an angle of 80° relative to the direction of the applied centrifugal force. The hybridization (detection) chamber 3 was located far from the center 23 of the rotor while the fragmentation chamber 4 was located close to the center of the rotor. After 1 min at 2000 rpm the liquid moved into the hybridization (detection) chamber 4 which contained the micro-array 16.

The device 10 was incubated in a PCR thermocycler (Eppendorf, Germany) for 1 hour at 60°C to allow the hybridization between the PCR product and the capture probes of the array. The temperature of the lid of the PCR thermocycler was set to 60°C.

After incubation the device 10 was centrifuged 1 min at 2000 rpm so that the liquid goes from hybridization (detection) chamber 3 to the fragmentation (detection) chamber 4.

The device 10 was then entered into the PortArray 5000™ Micro-array Reader (Aurora Photonics, Inc.). The reader was equipped with 532 nm laser. The full image of the array was collected in a single acquisition. The reader used an illumination system which transmits light through diffuser into the glass side, so that the light illuminated the array 16 from inside of the coverslip and produced evanescent field. The emission light was detected by a CCD camera. After image acquisition, the scanned 16-bit images were imported to the software, 'Genepix 5" (Axon, Union City, CA, USA) which was used to quantify the signal intensities. The signal was quantified on two capture probes P35S (SEQ ID NO: 1) and PAT1 (SEQ ID NO: 2) present in six replicates on the array.

The local background was subtracted and signal minus background was plotted against the number of cycles. The array 16 also contained capture probes for negative hybridization control and positive detection control labeled with Cy3 present in quadruplicate on the array.

The result showed a signal on the specific capture probe P35S. No signal was observed on capture probe PAT1. The hybridization control gave a positive signal on its capture probe TEPSPS1 (SEQ ID NO: 3).

## Claims

1. A device for a detection and/or quantification of a nucleotide sequence of an organism, said device comprising:
- at least one amplification chamber (1) for an amplification of the nucleotide sequence into amplified target nucleotide sequences, the said chamber having an inlet port (11) for an introduction into the amplification chamber of an amplification solution (1) comprising said nucleotide sequence and reagents for nucleotide sequence amplification, having a first outlet channel (13), and is made of solid material resistant to at least 90°C,
- at least one reagent chamber (2), said reagent chamber (2) having a second inlet port (12) for introduction of the reagent and a second outlet channel (14),
- a detection chamber (3) having fixed upon one of its solid support surface (15) at least one capture nucleotide probe being immobilized in discrete regions of said surface (15) to form a micro-array (16), said capture nucleotide probe being capable to bind by complementary base pairing with the amplified target nucleotide sequence or fragments thereof, wherein said surface (15) of the detection chamber (3) having fixed the capture nucleotide probe is maintained flat at temperature higher than 50°C, wherein the flatness tolerance of said surface (15) is less than 800 nm, when heated at 50°C and wherein said surface (15) has a light transmittance higher than 60% at wavelength between 400 and 600 nm and,
- a fragmentation chamber (4) for a fragmentation of amplified target nucleotide sequences into fragments, the said fragmentation chamber being connected to the amplification chamber (1) and to the reagent chamber (2) by the first and second outlet channels (13, 14) and being connected to the detection chamber (3) by a third outlet channel (19) and wherein said first and second channels (13, 14) are located at a different level than the third channel (19).

2. The device according to claim 1, wherein the amplification chamber (1) is sealed to allow liquid evaporation by less than 10 % of the solution present in the amplification chamber (1) after 35 amplification cycles.

3. The device according to claims 1 or 2, wherein the solid material is selected from the group consisting of glass, a polymer material and a mixture thereof.

4. The device according to the claim 3, wherein the polymer material is selected from the group consisting of: polycarbonate (PC), polyethylene (PE), Cycloolefin copolymer (COC), cyclic olefin polymer (COP), and a mixture thereof.

5. The device according to any of the preceding claims, which further comprises:
- one or more additional chamber(s) connected to one or two of the other chambers (1,2,3,4).

6. The device according to any of the preceding claims, wherein the capture nucleotide probe comprises a spacer portion and a capture portion being capable to bind by complementary base pairing with amplified target nucleotide sequences or their fragments.

7. The device of claim 6, wherein the spacer portion is a polynucleotide being at least about 20 nucleotides long.

8. The device according to any of the preceding claims, wherein the multiple capture nucleotide probes are immobilized upon the solid support surface (15) in the form of a micro-array (16) having at least four different discrete regions of the solid support surface (15)

9. The device according to any of the preceding claims, wherein the target nucleotide sequence is a nucleotide base or SNP.

10. The device of claim 6, wherein the capture nucleotide probe comprises:
- a capture portion of 10 to 100 nucleotides that is capable to bind by complementary base pairing with a specific sequence of the amplified target nucleotide sequences or their fragments such that said capture portion defines two non-complementary ends of the amplified target nucleotide sequences or their fragments and,
- a spacer portion having at least 20 nucleotides, and wherein the two non-complementary ends of the amplified target nucleotide sequences or their fragments comprise a spacer end and a non-spacer end, such that the spacer end is non-complementary to the spacer portion of the capture nucleotide probe, and said spacer end exceeds said non-spacer end by at least 50 bases.

11. The device according to any of the preceding claims, which further comprises at least one adhesive element or cap (29) for closing the first and the second inlet port(s)(11,12).

12. The device according to any of the preceding claims, wherein the detection chamber (3) is made of a solid material resistant to at least 95°C.

13. An automate for a detection and/or quantification of a nucleotide sequence of an organism, said automate comprising:
- the device (10) according to claim 1 to 12,
- a temperature control system adapted to perform an automated amplification process of the nucleotide sequence in the amplification chamber (1) and to perform an hybridization of amplified nucleotide sequence or their fragments in the detection chamber (3) of the device,
- one or more mean(s) to transfer a solution from the amplification chamber (1) and reagent chamber (2) to the detection chamber (3) or to the fragmentation chamber (4),
- a detector (43) for reading a signal resulting from a binding between the amplified target nucleotide sequence(s) or their fragments and their corresponding capture nucleotide molecules(s) fixed upon the solid support surface (15) of the detection chamber (3).

14. The automate of claim 13, wherein the transfer mean(s) allow(s) a solution transfer from the detection chamber (3) to the amplification chamber (1) and/or to the reagent chamber (2).

15. The automate of claim 13, wherein the transfer mean(s) allow(s) a solution transfer from the fragmentation chamber (4) to the detection chamber (3).

16. The automate of claim 13, wherein the transfer mean(s) allow(s) a solution transfer from the detection chamber (3) back to the fragmentation chamber (4).

17. The automate of claim 13, wherein the transfer mean is selected from the group consisting of a centrifugation force, a pressure force, a mechanical movement, an electric device or a mixture thereof,.

18. The automate of claims 13 to 17, wherein the reading detector (43) of the signal resulting from the binding between amplified target nucleotide sequence(s) or their fragments and capture nucleotide probe(s) allows a detection in presence of a solution containing the amplified target nucleotide sequence(s).

19. The automate of claims 13 to 17, wherein the reading detector (43) of the signal resulting from the binding between amplified target nucleotide sequence(s) or their fragments and capture nucleotide probe(s) allows a detection in absence of a solution containing the amplified target nucleotide sequence(s) (or their fragments)

20. The automate of claims 13 to 17, wherein the reading detector (43) of the signal resulting from the binding between amplified target nucleotide sequence(s) or their fragments and capture nucleotide molecule(s) comprises an evanescent field detector.

21. The automate of claim 20, wherein the evanescent field detector generates an incident light source (41) that illuminates a side (40) of the device (10).

22. The automate of claim 21, wherein the incident light source (41) is a non collimated laser source or a light emitting diode by a pair of optical fibber bundles.

23. The automate of claim 21 or 22, wherein the side (40) of the device forms an angle comprised between about 90° and 110° with the solid support surface (15) of the device (10).

24. The automate of claim 20, wherein the evanescent field detector generates an incident light source (41) that illuminates the solid support surface (15) of the device (10) with an incidence angle comprised between 10° and 90°.

25. The automate of claim 20, wherein the reading detector (43) allows a detection of an excited label of the amplified target nucleotide sequence(s), being excited by the evanescent field.

26. The automate of claim 25, wherein the detector (43) comprises a CCD camera.

27. The automate of claim 25, wherein the incident light source (41), the device (10) and the detector (43) are not moving relative to each other.

28. The automate of claims 21 to 38, wherein the temperature control system is a PCR thermocycler.

29. The automate of claims 13 to 27, wherein the temperature control system comprises a Peltier element.

30. The automate of claims 13 to 27, wherein the temperature control system comprises a thermoblock (32) of 96 wells.

31. The automate of claim 30, which further comprises a thermic adaptor (30) which is placed between the device (10) and the thermoblock (32) of the temperature control system and which is adapted to fit with the device (10) and the thermoblock (32).

32. The automate of claims 13 to 27, wherein the temperature control system comprises a thermoblock (32) made of at least 4 segments (33), each segment being adapted to receive a device (10) and wherein each segment (33) comprises a flat surface (34) and two cavities (35) adapted to receive the amplification chamber (1) and the reagent chamber (2) of the device (10).

33. A detection and/or quantification method of a nucleotide sequence of an organism, said method comprising the steps of:
- Introducing a solution comprising said nucleotide sequence through the inlet port (11) of the amplification chamber (1) of the device (10) or the automate according to any of the preceding claims 1 to 32,
- Submitting this nucleotide sequence to a nucleotide sequence amplification into amplified target nucleotide sequence
- Transferring a solution comprising the said amplified target nucleotide sequences or their fragments from the amplification chamber (1) or from the fragmentation chamber (4) into the detection chamber (3) of the device (10) or the automate,
- Putting into contact the solution comprising the amplified target nucleotide probes forming a micro-array (16) and being immobilized in discrete regions of the surface (15) of the solid support of the device (10) or the automate,
- Detecting and/or quantifying a signal resulting from the binding of the said target nucleotide sequences (or their fragments) to their corresponding capture probes at a discrete region upon the surface (15) of the solid support.

34. The method of claim 33, which further comprises the step of correlating the detected and/or quantified signal at the discrete region of the solid support surface (15) to the detection and/or quantification of the nucleotide sequence present in the biological sample.

35. The method of the claims 33 to 34, wherein the transferring step is obtained by a method selected from the group consisting of an use of a centrifugation force, an use of a pressure force, an use of a mechanical movement or an use of an electric device.

36. The method of claims 33 or 35, wherein the detection and/or quantification of the signal is obtained by a detection of an evanescent field generated by an incident light source (41) that illuminates a side (40) of the device (10), wherein the evanescent field preferably excites a label fixed upon the amplified target nucleotide sequence s or their fragments.


SEQUENCE LISTING

<110>  Eppendorf array technologies SA

<120>  Device and/or method for the detection of amplified nucleotides
       sequences on micro-arrays

<130>  EATE013B

<160>  7

<170>  PatentIn version 3.3

<210>  1
<211>  26
<212>  DNA
<213>  Synthetic primer TP35S

<400>  1
gtcatccctt acgtcagtgg agatat                                          26


<210>  2
<211>  25
<212>  DNA
<213>  Synthetic primer TPAT1

<400>  2
ctgtgtatcc caaagcctca tgcaa                                           25


<210>  3
<211>  23
<212>  DNA
<213>  Synthetic primer TEPSPS1

<400>  3
ctcctactcg ccgccctgtc cga                                             23


<210>  4
<211>  90
<212>  DNA
<213>  Synthetic primer Spacer


<220>
<221>  modified_base
<222>  (1)..(1)
<223>  5' Amine-adenosine

<400>  4
aaagttgagt ccatttgtga tgctagaaaa gttggaactt tcttgaacgt ctcctatatg     60

tcatacatga ataggttgat tttactgtac                                      90


<210>  5
<211>  22
<212>  DNA
<213>  Synthetic primer OP35SF


<220>
<221>  misc_feature
<222>  (1)..(1)
<223>  5'Cy3

<400> 5
cgtcttcaaa gcaagtggat tg 22

<210> 6
<211> 22
<212> DNA
<213> Synthetic primer OP35SR

<400> 6
tcttgcgaag gatagtggga tt 22

<210> 7
<211> 23
<212> DNA
<213> Synthetic primer positive control

<220>
<221> misc_feature
<223> 5' Cy3

<400> 7
tcggacaggg cggcgagtag gag 23

SEQUENCE LISTING

<110> Eppendorf array technologies SA

<120> Device and/or method for the detection of amplified nucleotides
sequences on micro-arrays

<130> EATE013B

<160> 7

<170> PatentIn version 3.3

<210> 1
<211> 26
<212> DNA
<213> Synthetic primer TP35S

<400> 1
gtcatccctt acgtcagtgg agatat 26

<210> 2
<211> 25
<212> DNA
<213> Synthetic primer TPAT1

<400> 2
ctgtgtatcc caaagcctca tgcaa 25

<210> 3
<211> 23
<212> DNA
<213> Synthetic primer TEPSPS1

<400> 3
ctcctactcg ccgccctgtc cga 23

<210> 4
<211> 90
<212> DNA
<213> Synthetic primer Spacer

<220>
<221> modified_base
<222> (1)..(1)
<223> 5' Amine-adenosine

<400> 4
aaagttgagt ccatttgtga tgctagaaaa gttggaactt tcttgaacgt ctcctatatg 60

tcatacatga ataggttgat tttactgtac 90

<210> 5
<211> 22
<212> DNA
<213> Synthetic primer OP35SF

<220>
<221> misc_feature
<222> (1)..(1)
<223> 5'Cy3

<400>  5
cgtcttcaaa gcaagtggat tg                                              22

<210>  6
<211>  22
<212>  DNA
<213>  Synthetic primer OP35SR

<400>  6
tcttgcgaag gatagtggga tt                                              22

<210>  7
<211>  23
<212>  DNA
<213>  Synthetic primer positive control

<220>
<221>  misc_feature
<223>  5' Cy3

<400>  7
tcggacaggg cggcgagtag gag                                             23